# EUROPEAN PATENT APPLICATION

(11) **EP 0 856 325 A2**
(43) Date of publication of application: **05.08.1998**
(21) Application number: 98300630.5
(22) Date of filing: 29.01.1998
(51) Int. Cl.: A61M 5/32

(54) **Needle disposal device**

(30) Priority: 29.01.1997 GB 9701803
(71) Applicant: Rinfret, John Henry Temple, Woodhall Spa, Lincolnshire LN10 6RY (GB); Rinfret, Elizabeth Margaret, Woodhall Spa, Lincolnshire LN10 6RY (GB)
(72) Inventor: Rinfret, John Henry Temple, Woodhall Spa, Lincolnshire LN10 6RY (GB); Rinfret, Elizabeth Margaret, Woodhall Spa, Lincolnshire LN10 6RY (GB)
(74) Representative: Bucks, Teresa Anne

(57) **Abstract**

Numerous problems exist concerning the disposal of used needles from hypodermic syringes and the like because of the risk they may be reused or cause injuries by accidentally jabbing into somebody (so called "needle stick injury"). A needle disposal device therefore comprises a body having a recess formed in one face thereof and a central shaft projecting from the recess, a pair of coaxial bores extending through the outer walls of the body into the recess and a third coaxial bore extending through the shaft. An annular cutting member is located on the shaft in the recess, the cutting member having a pair of coaxial bores extending therethrough and the cutting member being rotatable about the shaft to enable the bores therethrough to be aligned with the bores in the body to form a continuous needle receiving duct having a longitudinal axis which is perpendicular to the axis of rotation of the cutting member. The cutting member is further rotatable to disalign the bores and effect cutting of a needle in the duct at the junctions of the bores in the cutting member with the bores in the body. Container means having an aperture are provided communicating with the needle receiving duct for collecting cut needle parts.

## Description

Numerous problems exist concerning the disposal of used needles from hypodermic syringes and the like because of the risk they may be reused or cause injuries by accidentally jabbing into somebody (so called "needle stick injury").

Whilst modern needles are intended for a single use only, the overriding problem and fear is of a needle stick injury being caused by a needle which is infected with a serious disease.

In the medical and research industry, the usual method of disposing of waste sharp objects of all kinds is to collect them into a clearly labelled "sharps box". However, because these boxes are usually incinerated when full, they are often made relatively cheaply from plastic or cardboard and have comparatively thin walls. Consequently, it has been known for needles to pierce the walls of the box and then cause a needle stick injury. Another cause of needle stick injury is when these boxes are filled above their intended capacity and someone is jabbed by a needle when attempting to place further sharp objects into the box, or if a syringe falls awkwardly into a partially full box and points up at an angle.

To overcome these problems, a number of devices have been developed in the past. However, many of these have been concerned only with chopping the needle from its hub so that it cannot be reused. Examples of such devices are disclosed in the following patents: US 3,914,865, US 3,785,233, US 4,255,996 and US 4,404,881. The problem with these devices is that most of the long length of the needle remains intact and is therefore capable of continuing to cause a needle stick injury. Devices have been proposed which chop needles into a number of smaller portions. However, such devices are generally quite complex with a large number of different components and hence of significant cost. An example is the device disclosed in US 4,445,644.

One object of the present invention is to provide a needle disposal device which cuts a needle into a number of short pieces with the minimum of effort and which can also cut the nib from a syringe to ensure that both needle and, where applicable, syringe are destroyed and cannot be reused.

Another object of the invention is to provide a simple device with relatively few components which can be manufactured in a cost effective manner.

According to the present invention there is provided a needle disposal device comprising:
a body having a recess formed in one face thereof and a central shaft projecting from the recess, a pair of coaxial bores extending through the outer walls of the body into the recess and a third coaxial bore extending through the shaft;
an annular cutting member located on the shaft in the recess, the cutting member having a pair of coaxial bores extending therethrough and the cutting member being rotatable about the shaft to enable the bores therethrough to be aligned with the bores in the body to form a continuous needle receiving duct having a longitudinal axis which is perpendicular to the axis of rotation of the cutting member; the cutting member being further rotatable to disalign the bores and effect cutting of a needle in the duct at the junctions of the bores in the cutting member with the bores in the body; and
container means having an aperture communicating with the needle receiving duct for collecting cut needle parts.

Thus, the device essentially consists of two intermeshing components but enables a needle to be cut at four locations.

In a preferred embodiment, the diameter of the needle receiving duct is sufficiently large to allow the hub of a needle, which is the widened part which fits over the nib of a syringe, to be inserted into it, thereby allowing the nib to be severed from the syringe in order to destroy it, such that cutting of a needle occurs first at the junctions of the bores in the cutting member with the bores in the outer walls of the body and upon further rotation of the cutting member, subsequent cutting of a needle occurs at the junctions of the bores in the cutting member with the bore in the shaft.

Preferably, the annular recess in the body is closely spaced to the face of the body which defines an entrance to the needle receiving duct to ensure that a syringe nib can protrude into one of the bores defined in the cutting member.

If it is desired to cut a needle into a greater number of pieces, the body may comprise a plurality of coaxial annular recesses separated by coaxial annular walls and the cutting member may be formed with a plurality of coaxial annular ridges which are each locatable in a corresponding recess, wherein a pair of bores extend through each wall of the body and each ridge of the cutting member such that upon rotation of the cutting member all the bores are alignable to define the continuous needle receiving duct. In this way, there is a greater number of junctions between bores in the cutting member and bores in the body and so a greater number of cuts.

Advantageously, the entrance to the needle receiving duct is countersunk to provide a seat for receiving the end of a syringe to ensure the needle is inserted to the full extent into the device.

In a preferred embodiment, stop means are provided in order to limit the extent of rotation of the cutting member relative to the body.

The container means preferably comprises a hollow casing receiving a removable receptacle for collecting the broken needle parts and said syringe nibs. In this way, the receptacle can be removed when it is full, to be sealed and incinerated in the normal way.

The cutting member is preferably provided with a handle means to facilitate its rotation relative to the body.

Preferably, the body and the cutting means are manufactured from hardened stainless steel.

It is also preferable if means are provided to secure the cutting member against movement parallel to its axis of rotation in order to retain it within the recess in the body.

Preferably means are provided for selectively closing the aperture to the container means.

The invention will now be described in detail, by way of example only, with reference to the accompanying drawings in which:
Figure 1 is a front view of the disposal device in accordance with one embodiment of the present invention; and
Figure 2 is a cross-sectional view of the device of Figure 1 along the line A-A.

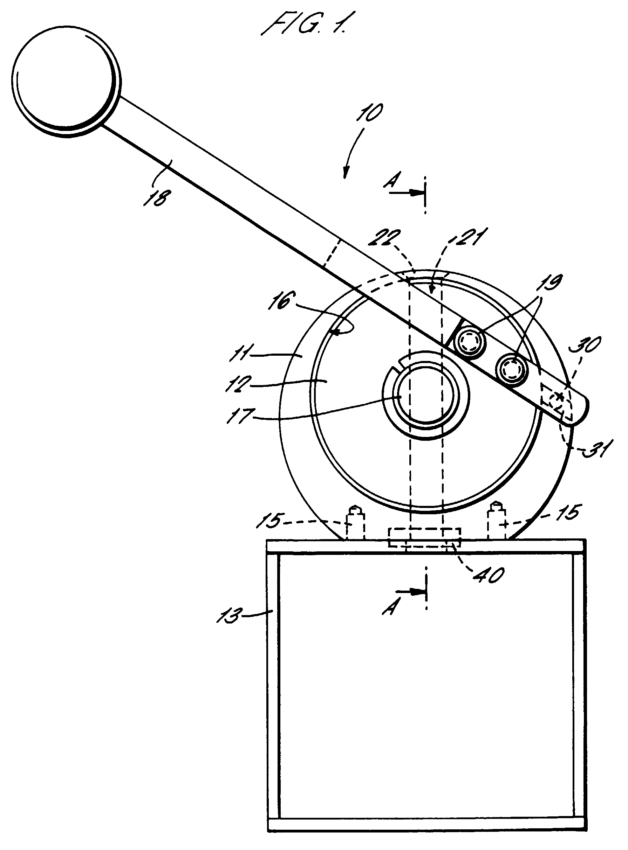

Referring to Figure 1, the needle disposal device 10 consists of two main components which mesh together. These are a body 11 and a cutting member 12. Both items are preferably manufactured from hardened stainless steel.

The body 11 is mounted on a hollow casing 13 which preferably receives a removable container (not shown) for collecting broken needles and severed syringe nibs. The body 11 may be secured to the casing 13 in any convenient manner and in this example it is secured by means of screws inserted through the casing into threaded holes 15 in the body 11.

One side face of the body 11 is provided with a deep recess 16 which has a central projecting spindle 17 projecting from it.

The cutting member 12 is an annular member dimensioned so as to fit on the spindle 17 and in the recess 16 with a close but sliding fit so that the cutter 12 is rotatable about the spindle 17 with an axis of rotation X-X. A handle or lever 18 is attached to the outwardly facing surface of the cutting member 12, by example by screws 19, to facilitate rotation of the cutting member 12. The angle at which the handle 18 is attached to the cutting member 12 may be different to that illustrated. For example, the handle 18 may be attached almost vertically so that it does not stick out to the side of the device. Alternatively the device 10 could be modified to include a Bowden cable to allow foot pedal operation.

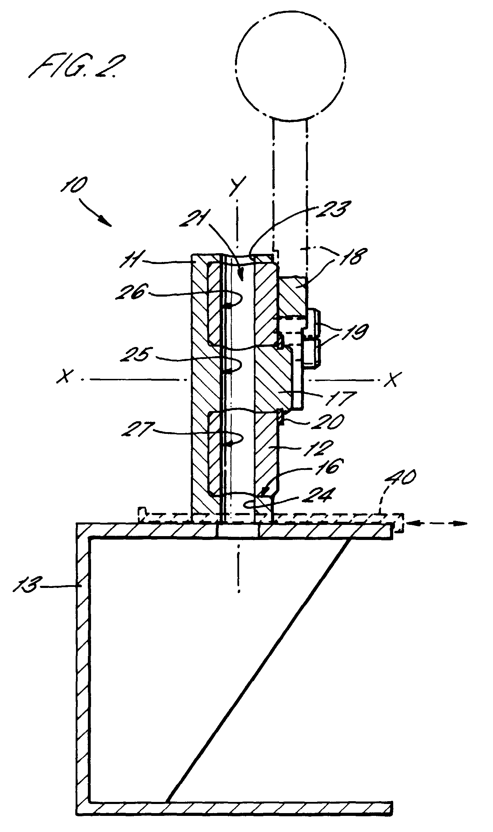

As seen in Figure 2, the spindle 17 preferably extends slightly outwardly of the cutting member 12 to allow the cutting member 12 to be secured within the body 11 and prevented from moving parallel to its axis of rotation X-X, for example by means of a circlip 20.

A needle receiving duct 21, which is preferably vertically aligned in use, is provided in the device 10 and is made up of a series of bores extending through the body 11, the spindle 17, the cutting member 12 and into the casing 13. The body 11 is provided with a pair of coaxial upper and lower bores 23,24 in the outer walls of the body 11 above and below the recess 16 and a third bore 25 extends through the spindle 17, coaxial with the bores 23,24. The cutting member 12 has a pair of coaxial bores 26,27 each extending between the outer and inner peripheries of the annulus. The casing 13 has an aperture 28 beneath the lower bore 24 in the body 11.

Therefore, the cutting member 12 can be rotated so as to align its bores 26,27 with the bores 23,24,25 in the body 11 to form the continuous needle receiving duct 21 extending from the top of the device 10 into the casing 13. The longitudinal axis Y-Y of the duct 21 is perpendicular to the axis of rotation X-X of the cutting member 12.

The device may be used to destroy any form of needles, especially those from hypodermic syringes and vacutainers (™), single or double ended, push-on or screwed needles.

The diameter of the needle receiving duct 21 is sufficiently wide that, in cases where the needle and syringe are to be destroyed, the nib of a syringe can also be inserted to allow the nib to be severed from the main part of the syringe as described below. The entrance to the duct 21 may be countersunk to provide a depression 22 in which the end of the syringe may be seated to ensure that the syringe nib (where applicable), and needle are inserted into the duct 21 to the full extent. Ensuring that the needle is totally encased before it is cut helps to ensure that the end does not fly out of the body 11 during cutting. Furthermore, the uppermost outer wall of the body 11 which defines the bore 23 is preferably relatively thin so as to allow an inserted syringe nib to protrude through into the uppermost bore 26 of the cutting member 12.

In a modified embodiment of the invention a latch plate 40 (shown in dotted lines on Figs. 1 and 2) is located beneath the duct 21 which has a bore 42. The latch plate 41 slides from side to side to open or close the aperture 28 in the body 11. In the open position with bore 42 aligned with the needle duct 21 and the aperture 28, the device will handle all forms of needles attached to syringes. In the closed position, with the bore 42 out of line with the needle duct 21 and the aperture 28, it is used for needles that are loose after having been ejected or unscrewed from syringes, vacutainers or the like.

A block having a coaxially aligned bore with the needle duct 21 may be positioned between the body 11 and latch plate 40 to effectively lengthen the duct 21 and help to ensure that the needles are wholly encased prior to destruction.

In order to operate the device, a syringe is positioned so that the needle is inserted all the way into the needle receiving duct 21 and the nib of the syringe passes into the uppermost bore 26 formed in the cutting member 12. If the syringe is to be destroyed, the latch plate 40 is left open and the lever 18 and cutting member 12 are rotated, for example through about 60 degrees. As the bores 26,27 which extend through the cutting member 12 are moved relative to the bores 23,24,25 which run through the body 11, the edges of the bores act as cutting edges so that the needle is sheared into four pieces and the nib of the syringe is severed from the main body of the syringe. In other words, cutting will occur at the junctions of the bores 26,27 in the cutting member 12 with the bores 23,24,25 in the body 11. Thus, the body 11 does provide a cutting action in conjunction with the cutting member 12 itself.

For needles that are to be detached from their syringes or vacutainers etc, the latch plate 40 is closed and the needle inserted up to the middle of the needle hub. The lever 18 is rotated just enough to apply pressure on the hub to grip it, so that the syringe, or other device to which it is attached, can be unscrewed or otherwise detached. The lever 18 is released, so that the needle can fall down the duct 21 until it hits the plate 40. The device is then operated as above.

Returning the lever 18 to its start position re-aligns all the bores 23-27 which form the needle receiving duct 21, thereby allowing the broken parts of the needles and any severed syringe nibs, to fall through aperture 28 into the container provided inside the casing 13. Where the latch plate 40 has been closed, this will obviously need to be opened to allow the debris to fall into the container. Once the container is full, it can be removed, closed with a lid and destroyed by incineration as usual.

Preferably, the device 10 is also provided with stop members (not shown) which limit the extent of rotation of the cutting member 12 and indicate when the cutting member 12 is positioned with its bores 26,27 aligned with those in the body 11 in order to define the continuous needle receiving duct 21. For example, the end of the handle 18 may be extended to overlap the face of the body 11 on the right-hand side of the cutting member as seen in Figure 1. A ball ended spring plunger 30 may then be fitted in a transverse bore in the end of the lever which engages in a groove 31 in the body 11 in order to determine when the device is in the start position with the needle receiving duct 21 ready for insertion of a needle.

The most commonly used needle in hospitals is know as a "green hubbed" needle which is approximately 55mm in length, or 60mm when taking into account the nib. The hub of the needle is a widened part which fits over the narrowed nib of a syringe. In order to accommodate such a needle, in a preferred embodiment of the invention the annular recess 16 has an outside diameter of approximately 56mm and is approximately 20mm deep. The spindle 17 has a diameter of approximately 16mm. Therefore, the annular cutting member 12 will also have an outer diameter of approximately 56mm and an inner diameter of approximately 16mm. The needle receiving duct 21 has a diameter of approximately 9mm so that a syringe nib as well as a needle itself can fit loosely within it. The uppermost outer wall of the body 11 which defines the bore 23 and the entrance to the needle receiving duct 21 is preferably only approximately 1.5mm thick.

Consequently, a needle will be cut into four pieces, two of approximately 20mm in length, one of approximately 16mm in length and the fourth piece being whatever remaining length of the needle projects through the lowermost bore 24.

Clearly the dimensions of the device 10 can be altered to suit the desired result, to cut a needle into different lengths and/or to cut at desired locations. In the example above the uppermost cut will severe the nib from the syringe but will not cut through the needle hub itself, since this does not embrace all of the nib. This reduces the cutting effort required and ensures that should the needle hub have been manufactured from a particularly hard material the device need not cut through the hub itself.

As mentioned previously, the needle receiving duct 21 is preferably vertically aligned in use as shown in Figures 1 and 2. This is because it has been found that medical staff generally prefer this arrangement to a horizontally oriented duct since they find greater time and care is required to insert a needle correctly into a horizontal opening. In addition, this arrangement means that the handle 18 moves in a vertical plane which users often prefer to a handle which moves back and forth in a horizontal plane because the former is easier to operate and does not stick out from the sides of the device to such an extent, thereby taking up less space and presenting less of a hazard. Furthermore, the device 10 can be mounted on a heavy base plate beneath the casing 13 allowing it to be stable but still to be portable if it is in a location which must periodically be cleared for cleaning purposes.

A further advantage of the present invention is that not only is the needle broken into several small pieces, but also that the effort required to break a needle into several pieces is minimised.

As discussed above, it is desirable to break a needle into several pieces in order to destroy it completely and so that a greater number of broken needles can be fitted into a disposable container of given size. However, if multiple cuts are made simultaneously the effort required by a user is increased. In the present case, there are four cutting locations but the actual cutting only occurs at two of these locations at a time. This is because of the rotational action of the cutting member 12 about the axis X-X perpendicular to the longitudinal axis Y-Y of the needle receiving duct 21 and the large diameter of the duct 21 relative to the diameter of a typical needle.

The needle receiving duct 21 is made considerably larger than the diameter of a typical needle because it is desired to use the device to sever the syringe nib from the syringe as well as to break the needle. Therefore, the syringe nib must also fit in the bore 21. As the cutting member 12 is rotated, the edges of bores 26 and 27 at the outer periphery of the cutting member 12 are the first to come into contact with the needle. These cooperate with the edges of the bores 23 and 24 at the point where they enter the recess 16 to cut the needle at these two locations. Only upon further rotation of the cutting member 12 will the edges of bores 26 and 27 at the inner periphery of the cutting member 12 come into contact with and cut the needle in cooperation with the edges of bore 25 in the spindle 17. In other words, the total cutting action of the device is staggered and therefore the total force required to break the needle into four pieces is not required all at once. An additional advantage of minimising the force required is that the handle 18 need not be especially long to provide sufficient leverage. Therefore, the overall space envelope occupied by the device is reduced.

Thus, the present invention provides a simple device with very few components but which still cuts a needle into several small lengths with relative ease. In this way, reuse of the needle and the syringe are prevented, the risk of needle stick injury is reduced and the waste is collected in a more space efficient manner.

It will be apparent to a person skilled in the art that a number of modifications may be made to the syringe needle disposal device of the present invention. For example, in order to cut a needle into a greater number of pieces, the body 11 may be formed with a series of coaxial annular recesses and the cutting member 12 may be formed with a corresponding series of coaxial annular ridges which each fit into a respective recess to allow the body and the cutting member to mesh together. As before, the needle receiving duct will consist of a series of aligned apertures extending through the body and the cutting member, but in this case a greater number of cuts will be achieved. For ease of manufacture, the cutting member may be produced as a number of separate sector shaped pieces which can be fitted together with an appropriate bush to form an annular cutting member.

## Claims

1. A needle disposal device comprising:
a body having a recess formed in one face thereof and a central shaft projecting from the recess, a pair of coaxial bores extending through the outer walls of the body into the recess and a third coaxial bore extending through the shaft;
an annular cutting member located on the shaft in the recess, the cutting member having a pair of coaxial bores extending therethrough and the cutting member being rotatable about the shaft to enable the bores therethrough to be aligned with the bores in the body to form a continuous needle receiving duct having a longitudinal axis which is perpendicular to the axis of rotation of the cutting member; the cutting member being further rotatable to disalign the bores and effect cutting of a needle in the duct at the junctions of the bores in the cutting member with the bores in the body; and
container means having an aperture communicating with the needle receiving duct for collecting cut needle parts.

2. A needle disposal device as claimed in claim 1, wherein the diameter of the needle receiving duct is sufficiently large to allow a needle hub to be inserted into it, such that cutting of a needle occurs first at the junctions of the bores in the cutting member with the bores in the outer walls of the body and upon further rotation of the cutting member, subsequent cutting of a needle occurs at the junctions of the bores in the cutting member with the bore in the shaft.

3. A needle disposal device as claimed in claim 1 or claim 2, wherein the annular recess is closely spaced to the face of the body which defines an entrance to the needle receiving duct to allow a syringe nib to which a needle is attached to protrude into one of the bores defined by the cutting member.

4. A needle disposal device as claimed in any preceding claim, wherein the body further comprises a plurality of coaxial annular recesses separated by coaxial annular walls and the cutting member comprises a plurality of coaxial annular ridges each locatable in a corresponding recess, wherein a pair of coaxial bores extend through each wall of the body and each ridge of the cutting member such that upon rotation of the cutting member all the bores are alignable to define the continuous needle receiving duct.

5. A needle disposal device as claimed in any preceding claim, wherein the entrance to the needle receiving duct is countersunk to provide a seat for receiving the end of a syringe to which a needle is attached.

6. A needle disposal device as claimed in any preceding claim, further comprising stop means to limit the extent of rotation of cutting member relative to the body.

7. A needle disposal device as claimed in any preceding claim, wherein the container means comprises a hollow casing receiving a removable receptacle for collecting broken needle parts.

8. A needle disposal device as claimed in any preceding claim, wherein the cutting means is provided with a handle to facilitate rotation of the cutting means relative to the body.

9. A needle disposal device as claimed in any preceding claim, wherein the body and the cutting means are made from hardened stainless steel.

10. A needle disposal device as claimed in any preceding claim, further comprising means to secure the cutting means against movement parallel to its axis of rotation in order to retain it within the recess.

11. A needle disposal device as claimed in any preceding claim further comprising means for selectively closing the aperture to the container means.
